Europäisches Patentamt

European Patent Office

Office européen des brevets

(19)

(11) Publication number: **0 039 321**
**B1**

(12) **EUROPEAN PATENT SPECIFICATION**

(45) Date of publication of patent specification: **25.07.84**

(51) Int. Cl.³: **G 01 N 25/56**

(21) Application number: **81850077.9**

(22) Date of filing: **29.04.81**

(54) **Method of determining the mean moisture ratio of drying timber.**

(30) Priority: **30.04.80 SE 8003281**

(43) Date of publication of application:
**04.11.81 Bulletin 81/44**

(45) Publication of the grant of the patent:
**25.07.84 Bulletin 84/30**

(84) Designated Contracting States:
**AT BE CH DE FR GB IT LI LU NL**

(56) References cited:
**CH - A - 265 555**
**DE - A - 2 347 623**
**GB - A - 715 798**
**GB - A - 910 360**
**GB - A - 1 030 171**
**US - A - 3 350 789**

(73) Proprietor: **HB MEGARON**
**Lasarettsvägen 19**
**S-931 32 Skelleftea (SE)**

(72) Inventor: **Björkman, Erik**
**Mättsund 6062**
**S-951 90 Lulea (SE)**

(74) Representative: **Onn, Thorsten et al,**
**AB STOCKHOLMS PATENTBYRA Box 3129**
**S-103 62 Stockholm (SE)**

Courier Press, Leamington Spa, England.

## Description

This invention relates to a method of determining the mean moisture ratio of timber at one or more predetermined time/s during its drying process.

One prerequisite for being able to correctly control the drying of timber is to know the absolute moisture ratio of the timber. Heretofore, however, great difficulties were involved for obtaining this knowledge. At a normal drying process comprising the phases of heating, predrying, after-drying, cooling and conditioning, the possibilities of obtaining a correct value of the absolute moisture ratio are minimum. The reason of this is that the drying must proceed very carefully in order not to damage the timber, for example by water too rapidly evaporating from the timber which unavoidably would result in crack formation. A further reason is that the evaporation from moist timber depends on several more factors than on the moisture ratio of the timber.

The moisture ratio in timber, as known, varies in response to the temperature and relative humidity of the drying air, due to the fact, that moisture is permitted to evaporate from the timber until the drying air is not capable any longer to absorb additional water and to emit heat. This capacity of the drying air ceases when the moisture ratio of the timber has achieved a break-even point corresponding to the temperature and relative humidity of the drying air, i.e. hygroscopic equilibrium.

The amount of water evaporating from the timber can be determined by measuring, for example, the dry and the wet temperature of the air surrounding the timber when drying, i.e. of the drying air or drying climate, whereby also a value of the change per time unit of the moisture ratio of the timber is obtained. The method according to the invention of determining the mean moisture ratio of timber during its drying is based on these known conditions.

As a statement of the technological background reference is made to the prior art document US—A—3 350 789 and GB—A—1 030 171 relating to the determination of the moisture content of thin porous materials and more particularly to the continuous determination of the moisture content of such material while they are in motion in the course of processing, and to a moisture meter for use in a drying machine for sheet material, respectively.

The method according to the invention is characterized in that the drying climate established for said drying process is disturbed during a certain limited period following said predetermined time/s, and that the values of the dry and wet temperature variations of the drying climate both during and after the disturbance/s are recorded, and said values are processed in a calculation unit according to a predetermined mathematical evaluation pattern.

A disturbance introduced into or generated in the drying climate according to the invention may have the form of a substantial increase and/or decrease of the temperature of the drying climate, a sudden change of the ventilation cycle and/or of the drying air rate, a rapid change of the relative humidity, for example by supply of water, so-called steaming, or a combination of two or more of these forms. The disturbance, for example, may be a combination of rapid cooling and directly subsequent rapid heating while simultaneously the ventilation (amount of spent drying air ventilated per time unit) and the air rate are changed.

During the disturbance of the drying climate as well as after the same, the temperature progress for both the wet and the dry temperature in the drying air is recorded, and the values thus recorded then are processed, for example in a minicomputer after a predetermined mathematic pattern. This pattern includes one part concerning the generation of the disturbance and one evaluation part. For the evaluation, the time progress both for the disturbance and the response is observed, whereby a.o. the following variables are followed; absolute moisture content in the drying air—temperature drop through the timber charge in one or more places—dry and wet temperature—air rate through the timber charge—possibly supplied and removed energy.

For achieving an excellent result of the drying of timber, at least the mean ingoing moisture ratio, i.e. the moisture ratio of the timber after the heating phase of the drying process, and the mean moisture ratio after the cooling phase of the drying process must be known for being able to control at optimum the drying process.

As regards the determination of the mean ingoing moisture ratio, the disturbance must be commenced first after possible natural reactions caused, for example, by frozen timber and/or snow on the timber or by non-uniform condensation have decayed during the first phase of the heating, and after the drying climate has stabilized about a certain psychometer difference and to a substantially constant evaporation rate. First thereafter, thus, the disturbance may be commenced for generating necessary values for calculating the ingoing moisture ratio of the timber.

The determination of the mean final moisture ratio according to the invention is to be carried out in connection with the transition to the conditioning phase of the drying process, which transition per se implies a substantial disturbance of the drying climate. In said last mentioned phase of the drying process the timber can be subjected to relatively careless treatment without damage being caused thereon, but on the other hand the timber reacts very slowly on a changed climate during said phase. By introducing a disturbance into the drying climate, according to the principles on which the present invention is based, this disturbance is added to the one which automatically arises at the transition to the conditioning phase. Said introduced disturbance may be one of the aforesaid forms, a combination thereof or a substantial increase of the psychometer difference for several hours before the wet temperature is increased rapidly, for example by controlled steaming.

As regards the mathematic patterns for calculating the mean ingoing and final moisture ratio, respectively, they have different forms, but the controlled disturbance and the evaluation of the process response are the same in both cases, and also for the determination of the mean moisture ratio in the second phase of the drying process.

The principle, on which the present invention is based, can be utilized for different driers and for different ways of controlling the drying process and, in principle, during each phase of the drying process.

## Claims

1. A method of determining the mean moisture ratio of timber at one or more predetermined time/s during its drying process, characterized in that the drying climate established for said drying process is disturbed during a certain limited period following said predetermined time/s, and that the values of the dry and wet temperature variations of the drying climate both during and after the disturbance/s are recorded, and said values are processed in a calculation unit according to a predetermined mathematical evaluation pattern.

2. A method as defined in claim 1, characterized in that the or each disturbance of the drying climate is effected by a substantial increase and/or decrease of the dry temperature of the drying climate, change of the ventilation, air rate and/or psychrometer difference, and/or steaming.

3. A method as defined in claim 1 or 2, in which the drying process includes a heating phase characterized in that the or each disturbance of the drying climate is commenced after possible natural reactions caused, for example, by frozen timber, snow or non-uniform condensation have decayed and after the drying climate has stabilized about a certain psychrometer difference and at a substantially constant evaporation rate, to give the mean moisture ratio of the timber after the heating phase.

4. A method as defined in claim 1 or 2, characterized in that the or each disturbance of the drying climate is commenced at the transition to the conditioning phase of the drying process to give the mean final moisture ratio of the timber.

5. A method as defined in claim 4, characterized in that the or each disturbance consists of an increase of the psychrometer difference during some hours before the wet temperature of the drying climate is increased rapidly by, for example, controlled steaming.

## Patentansprüche

1. Verfahren zur Bestimmung des mittleren Feuchtigkeitsverhältnisses von Holz an einem oder mehreren vorbestimmten Zeitpunkten während seines Trocknungsprozesses, dadurch gekennzeichnet, daß das für genannten Trocknungsprozeß geschaffene Trocknungsklima während einer gewissen begrenzten, auf die genannte vorbestimmte Zeit oder Zeiten folgende Periode gestört wird, die Werte der trockenen und nassen Temperaturschwankungen des Trocknungsklimas sowohl während als auch nach der oder den Störungen aufgezeichnet werden, und genannte Werte in einer Recheneinheit gemäß einem vorbestimmten mathematischen Auswertungsmuster bearbeitet werden.

2. Verfahren nach Anspruch 1, dadurch gekennzeichnet, daß die oder jede Störung des Trocknungsklimas durch eine beträchtliche Erhöhung und/oder Senkung der Trockentemperatur des Trocknungsklimas, Änderung der Lüftung, des Luftflusses und/oder Psychrometerunterschiedes, und/oder Dämpfung bewirkt wird.

3. Verfahren nach Anspruch 1 oder 2, bei dem der Trocknungsprozeß eine Erwärmungsphase einschließt, dadurch gekennzeichnet, daß die oder jede Störung des Trocknungsklimas begonnen wird, nachdem mögliche natürliche Reaktionen, die z.B. durch gefrorenes Holz, Schnee oder ungleichmäßige Kondensation verursacht sind, abgeklungen sind, und nachdem das Trocknungsklima sich um einen gewissen Psychrometerunterschied, bei einer im wesentlichen konstanten Verdampfungsgeswindigkeit stabilisiert hat, um das mittlere Feuchtigkeitsverhältnis nach der Erwärmungsphase zu ergeben.

4. Verfahren nach Anspruch 1 oder 2, dadurch gekennzeichnet, daß die oder jede Störung des Trocknungsklimas beim Übergang zur Konditionierungsphase des Trocknungsprozesses begonnen wird, um das endgültige Feuchtigkeitsverhältnis des Holzes zu ergeben.

5. Verfahren nach Anspruch 4, dadurch gekennzeichnet, daß die oder jede Störung aus einer Erhöhung des Psychrometerunterschiedes während einiger Stunden besteht, bevor die Naßtemperatur des Trocknungsklimas rasch, z.B. durch geregelte Dämpfung, erhöht wird.

## Revendications

1. Procédé de détermination du taux d'humidité moyen de bois à un ou plusieurs moments prédéterminés au cours du processus de séchage du bois, caractérisé en ce que le climat de séchage établi en vue dudit processus de séchage est perturbé pendant une certaine période limitée qui suit le(s) dit(s) moment(s) prédéterminé(s), que les valeurs des variations de la température au thermomètre sec et mouillé du climat de séchage, avant et après la (les) perturbation(s), sont enregistrées, et que lesdites valeurs sont traitées dans une unité de calcul conformément à un modèle prédéterminé d'évaluation mathématique.

2. Procédé selon la revendication 1, caractérisé en ce que chaque perturbation du climat de séchage est effectuée par une augmentation et/ou baisse considérables de la température au thermomètre sec du climat de séchage, une modification de la ventilation, du débit d'air et/ou de la différence psychrométrique, et/ou par passage à la vapeur.

3. Procédé selon l'une des revendications 1 et 2, dans lequel le processus de séchage comprend une phase de chauffage, caractérisé en ce que chaque perturbation du climat de séchage est commencée après déclin des réactions naturelles éventuelles dues, par exemple, à l'état gelé du bois, à la neige ou à la condensation non uniforme, et après stabilisation du climat de séchage autour d'une différence psychrométrique donnée et à un taux d'évaporation sensiblement constant, afin de fournir le taux d'humidité moyen du bois à la suite de la phase de chauffage.

4. Procédé selon l'une des revendications 1 et 2, caractérisé en ce que chaque perturbation du climat de séchage est commencée à la transition à la phase de conditionnement du processus de séchage, afin de fournir le taux d'humidité final moyen du bois.

5. Procédé selon la revendication 4, caractérisé en ce que chaque perturbation consiste en une augmentation de la différence psychrométrique pendant quelques heures avant que la température au thermomètre mouillé du climat de séchage soit augmentée rapidement, par exemple par passage à la vapeur contrôlé.